(19) 

**Europäisches Patentamt**
**European Patent Office**
**Office européen des brevets**

(11) **EP 4 781 917 A1**

(12) # EUROPEAN PATENT APPLICATION
published in accordance with Art. 153(4) EPC

(43) Date of publication:
**29.07.2026 Bulletin 2026/31**

(21) Application number: **23953172.6**

(22) Date of filing: **22.09.2023**

(51) International Patent Classification (IPC):
**A61B 8/08** (2006.01)    **G06T 7/62** (2017.01)

(52) Cooperative Patent Classification (CPC):
**A61B 8/08; G06T 7/62**

(86) International application number:
**PCT/KR2023/014572**

(87) International publication number:
**WO 2025/063347 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.09.2023 KR 20230123898**

(71) Applicant: **Mcube Technology Co., Ltd.
Seoul 07789 (KR)**

(72) Inventors:
• **CHO, Min Hyoung
Seongnam-si Gyeonggi-do 13499 (KR)**
• **CHO, Hyun Hwa
Hanam-si Gyeonggi-do 13015 (KR)**
• **SUH, Jae Joon
Suwon-si Gyeonggi-do 16704 (KR)**

(74) Representative: **Engelhard, Markus
Boehmert & Boehmert
Anwaltspartnerschaft mbB
Pettenkoferstrasse 22
80336 München (DE)**

(54) **BLADDER VOLUME MEASURING SYSTEM AND BLADDER VOLUME MEASURING METHOD IN SYSTEM**

(57)    The present invention relates to a bladder volume measurement system and a bladder volume measurement method. The bladder volume measurement method includes: calculating, by using a predetermined region-of-interest extraction algorithm, a probability value that each unit region constituting an ultrasound image is included in a region of interest; determining weights for the probability values of the respective unit regions constituting the ultrasound image by using a preset weighting function; and calculating and providing an area or a volume of the region of interest by using the probability values and the weights for all unit regions constituting the ultrasound image.

[FIG. 5]

START

Generating a fan-shaped two-dimensional ultrasound image using the ultrasound signals — S500

Obtaining probability values for unit regions constituting of the ultrasound image by using a region-of-interest extraction algorithm — S510

Assigning a weight for each probability value by using a weighting function having a first threshold value and a second threshold value — S520

Obtaining a weighted area or a weighted volume of each unit region by using the probability value and the weight corresponding to each unit region of the ultrasound image — S530

Summing the weighted areas or the weighted volumes of all unit regions of the ultrasound image to obtain a total area or a total volume of the region of interest — S540

END

**Description**

[TECHNICAL FIELD]

The present invention relates to a bladder volume measurement system. More specifically, the present invention relates to a bladder volume measurement system and a bladder volume measurement method in the system configured to extract a bladder region from an ultrasound image using a region-of-interest extraction algorithm and to measure a bladder volume by using probability values for respective pixels of the ultrasound image obtained as an extraction result and weights according to a preset weighting function, thereby enabling the bladder volume to be measured more accurately.

The present invention was supported by the Korea Medical Device Development Fund, funded by the Ministry of Science and ICT, the Ministry of Trade, Industry and Energy, the Ministry of Health and Welfare, and the Ministry of Food and Drug Safety of the Republic of Korea (Project No.: 1711194192, RS-2020-KD000043).

[BACKGROUND ART]

In general, an ultrasound system is a system for examining an internal state of an object. The ultrasound system transmits an ultrasound signal to an object to be examined by means of a piezoelectric effect of a probe, i.e., a transducer, receives an ultrasound signal reflected and returned from a discontinuous surface of the object, converts the received ultrasound signal into an electrical signal, and outputs the electrical signal to a predetermined imaging device. Such ultrasound systems are widely used in medical diagnosis, non-destructive testing, underwater exploration, and the like. In particular, by extracting a region of interest, such as a specific organ or lesion, from a medical image acquired using the ultrasound system and measuring a size of the extracted region of interest, a technique for determining or diagnosing a condition of a patient has been widely used.

Meanwhile, in examinations for bladder abnormalities or voiding dysfunction, measuring the amount of urine in the bladder is used as an essential factor. In addition, in order to prevent urinary retention that may occur after surgery, the amount of urine in the bladder is measured prior to urination using a catheter, and in voiding training, the amount of urine in the bladder is also measured and used as a guideline. The amount of urine filled in the bladder is equal to the volume of the bladder. Accordingly, the amount of urine in the bladder may be measured by measuring the volume of the bladder. Further, an ultrasound scanner may be used to measure the volume of the bladder.

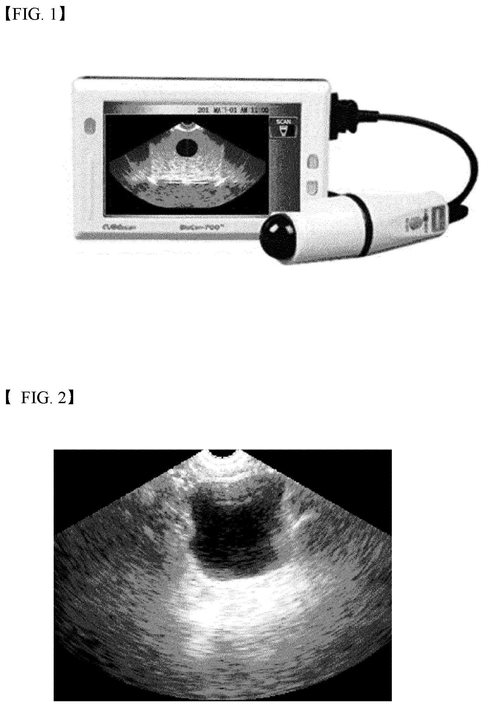

As described above, the ultrasound scanner for measuring the amount of urine in the bladder acquires ultrasound signals corresponding to fan-shaped scan planes including the bladder, obtains two-dimensional or three-dimensional ultrasound images from the ultrasound signals for the scan planes, extracts a bladder region, which is a region of interest, from the ultrasound images, calculates an area or a volume of the bladder using information on the extracted bladder regions, and estimates the amount of urine in the bladder based thereon. FIG. 1 illustrates an example of a bladder volume measurement system using an ultrasound scanner, and FIG. 2 illustrates an example of a two-dimensional ultrasound image obtained using the bladder volume measurement system of FIG. 1.

Various algorithms for extracting a bladder region, which is a region of interest, from two-dimensional or three-dimensional ultrasound images have been proposed. An algorithm for extracting a region of interest from ultrasound images may use an artificial intelligence model and a machine learning algorithm, or may extract a desired region of interest by analyzing a magnitude or intensity of ultrasound signals.

However, since a dedicated ultrasound scanner for bladder volume measurement is manufactured in a small-sized or portable form to be easily movable, ultrasound images obtained by the dedicated ultrasound scanner generally have a low resolution. Accordingly, due to the low resolution of images acquired by the dedicated ultrasound scanner for bladder volume measurement, pixels or voxels have a relatively large size. Due to such characteristics, not only does a partial volume effect occur in the ultrasound images, but also boundaries of a region of interest or lesions become ambiguous. In addition, noise and artifacts generated by the ultrasound scanner for acquiring medical images frequently occur. Accordingly, there is a problem in that it is generally difficult to accurately extract a region of interest from a low-resolution ultrasound medical image. Furthermore, due to such problems, it also becomes difficult to accurately measure an area or a volume of the bladder from the region of interest extracted from the ultrasound image.

Meanwhile, among the aforementioned region-of-interest extraction algorithms, when a machine learning algorithm is used, a probability value indicating that each pixel or each preset unit region of the ultrasound image is included in the region of interest is provided. The region of interest is then finally extracted from the ultrasound image by binarizing the probability values obtained according to the extraction algorithm. Even if the result according to the extraction algorithm is not provided in the form of a probability, a magnitude of a pixel value may be used during a calculation process. In this case, when normalized by the largest pixel value, the result may be regarded as a type of probability value.

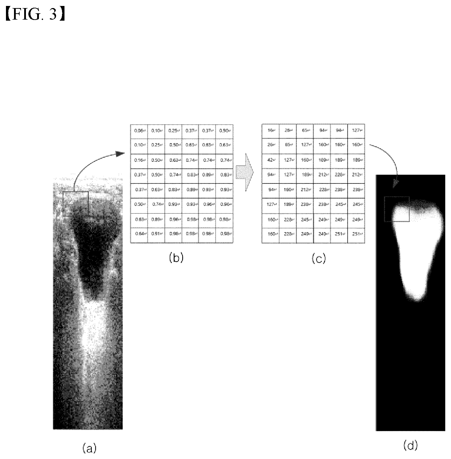

FIG. 3(a) is a two-dimensional ultrasound image acquired using a bladder volume measurement system, FIG. 3(b) illustrates a portion of probability values output for respective pixels of the two-dimensional ultrasound image shown in FIG. 3(a) using a machine learning algorithm, FIG. 3(c) shows the probability values displayed in FIG. 3(b) converted into pixel values, and FIG. 3(d) shows an image reconstructed using the pixel values displayed in FIG. 3(c). As illustrated in FIG. 3(b), the output probability values have values between 0.0 and 1.0, and when the probability is 1.0, it indicates that the corresponding pixel has a 100% probability of being part of the bladder. FIG. 3(d) is an image reconstructed by converting the probability values of 0.0 to 1.0 for respective pixels of the ultrasound image shown in FIG. 3(a) into pixel values ranging from 0 to 255.

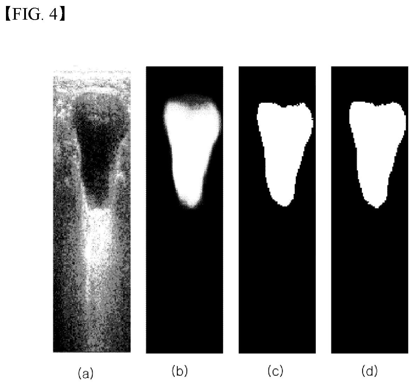

In a conventional technique, in order to obtain an area or a volume of a bladder, which is a region of interest, from an ultrasound image, a binarization method for detecting a boundary from an image such as that shown in FIG. 4(a) is used. The binarization method separates a background region and a region of interest using a preset threshold. FIG. 4(a) is a two-dimensional ultrasound image, FIG. 4(b) is an image reconstructed from probability values output by an extraction algorithm, FIG. 4(c) is an image binarized by setting a threshold to 0.5, and FIG. 4(d) is an image binarized by setting a threshold to 0.4. In the case of FIG. 4(c), the number of pixels having probability values equal to or greater than the threshold is 2,554, and in the case of FIG. 4(d), the number of pixels having probability values equal to or greater than the threshold is 2,661. As illustrated in FIG. 4, the position of a boundary of the region of interest varies depending on the threshold, and the extracted region of interest also varies accordingly. Therefore, when different thresholds are used, the extracted region of interest differs, and thus the estimated area or volume of the region of interest also differs. In other words, when the binarization method is used, the area or volume of the region of interest changes very sensitively depending on the threshold set for binarization. Accordingly, the setting of the threshold has a significant influence on the reliability of a final result value.

[DETAILED DESCRIPTION OF THE INVENTION]

[TECHNICAL PROBLEM]

In order to solve the above-described problems, an object of the present invention is to provide a bladder volume measurement system and a bladder volume measurement method configured to extract a region of interest by applying a region-of-interest extraction algorithm to an ultrasound image, and to accurately measure an area or a volume of the region of interest by using probability values for respective pixels output as an extraction result and weights according to a preset weighting function based on a distribution of the probability values.

[TECHNICAL SOLUTION]

A bladder volume measurement system according to a first aspect of the present invention for achieving the above-described technical objective comprises: an ultrasound probe configured to receive ultrasound signals for acquiring an ultrasound image by using an ultrasound transducer; and a controller configured to measure and provide an area or a volume of a region of interest included in the ultrasound signals received from the ultrasound probe, wherein the controller generates the ultrasound image by using the ultrasound signals received from the ultrasound probe, infers, by using a preset region-of-interest extraction algorithm, a probability value that each unit region constituting the ultrasound image is included in the region of interest, assigns a weight by using a preset weighting function according to a distribution of the probability values, and calculates and provides the area or the volume of the region of interest by using the probability values and the weights for all unit regions constituting the ultrasound image.

In the bladder volume measurement system according to the first aspect described above, it is preferable that the controller assign weights for unit regions constituting the ultrasound image by using the weighting function, multiply the weight of each unit region by a unit area or a unit volume of the corresponding unit region to obtain a weighted area or a weighted volume of each unit region, and calculate the area or the volume of the region of interest by summing the weighted areas or the weighted volumes of all unit regions constituting the ultrasound image.

In the bladder volume measurement system according to the first aspect described above, it is preferable that the weighting function includes a first threshold value and a second threshold value, assign a preset minimum weight to probability values smaller than the first threshold value, assign a preset maximum weight to probability values greater than the second threshold value, and assign a weight, linearly or nonlinearly within a range between the minimum weight and the maximum weight, to probability values between the first threshold value and the second threshold value.

In the bladder volume measurement system according to the first aspect described above, it is preferable that the minimum weight is "0" and the maximum weight is "1".

A bladder volume measurement method according to a second aspect of the present invention is a bladder volume measurement method performed by a controller of a bladder volume measurement system configured to measure and provide a volume of a bladder by using ultrasound signals, the method comprising: (a) generating an ultrasound image by using ultrasound signals received from an ultrasound probe; (b) inferring, by using a preset region-of-interest extraction algorithm, a probability value that each unit region constituting the ultrasound image is included in a region of interest; and (c) assigning weights for the respective unit regions constituting the ultrasound image by using a preset weighting function, calculating and providing an area or a volume of the region of interest by using the probability values and the weights for all unit regions constituting the ultrasound image.

In the bladder volume measurement method according to the second aspect described above, it is preferable that step (c) comprises: (c1) assigning weights for unit regions constituting the ultrasound image by using the weighting function; (c2) multiplying the weight of each unit region by a unit area or a unit volume of the corresponding unit region to obtain a weighted area or a weighted volume of each unit region; and (c3) calculating the area or the volume of the region of interest by summing the weighted areas or the weighted volumes of all unit regions constituting the ultrasound image.

In the bladder volume measurement method according to the second aspect described above, it is preferable that the weighting function includes a first threshold value and a second threshold value, assign a preset minimum weight to probability values smaller than the first threshold value, assign a preset maximum weight to probability values greater than the second threshold value, and assign a weight, linearly or nonlinearly within a range between the minimum weight and the maximum weight, to probability values between the first threshold value and the second threshold value.

A bladder volume measurement system according to a third aspect of the present invention comprises: an ultrasound probe configured to receive ultrasound signals for acquiring an ultrasound image by using an ultrasound transducer; and a controller configured to measure and provide an area of a region of interest included in the ultrasound signals received from the ultrasound probe, wherein the controller analyzes the ultrasound signals received from the ultrasound probe to detect a front wall point and a rear wall point of the bladder, sets a front wall boundary region including a preset anterior-posterior region of the front wall point of the bladder, sets a rear wall boundary region including a preset anterior-posterior region of the rear wall point of the bladder, assigns weights for respective positions of the ultrasound signals based on the front wall boundary region and the rear wall boundary region, and calculates and provides an area or a volume of a bladder region by using the weights for the respective positions of the ultrasound signals.

In the bladder volume measurement system according to the third aspect described above, it is preferable that the controller sets a weight for positions prior to a start point of the front wall boundary region to a preset minimum weight, sets a weight for each position within the front wall boundary region based on a difference in ultrasound signal intensity between a start point and an end point of the front wall boundary region, sets a weight for each position between the front wall boundary region and the rear wall boundary region to a preset maximum weight, sets a weight for each position within the rear wall boundary region based on a difference in ultrasound signal intensity between a start point and an end point of the rear wall boundary region, and sets a weight for positions after an end point of the rear wall boundary region to a preset minimum weight.

A bladder volume measurement method according to a fourth aspect of the present invention is a bladder volume measurement method performed by a controller of a bladder volume measurement system configured to measure and provide a volume of a bladder by using ultrasound signals, the method comprising the following steps: (a) analyzing ultrasound signals received from an ultrasound probe to detect a front wall point and a rear wall point of the bladder; (b) setting a front wall boundary region including a preset anterior-posterior region of the front wall point of the bladder, and setting a rear wall boundary region including a preset anterior-posterior region of the rear wall point of the bladder; (c) assigning weights for respective positions of the ultrasound signals based on the front wall boundary region and the rear wall boundary region; and (d) calculating and providing an area or a volume of a bladder region by using the weights for the respective positions of the ultrasound signals.

In the bladder volume measurement method according to the fourth aspect described above, it is preferable that step (c) comprises: (c1) setting a weight for positions prior to a start point of the front wall boundary region to a preset minimum weight; (c2) setting a weight for each position within the front wall boundary region based on a difference in ultrasound signal intensity between a start point and an end point of the front wall boundary region; (c3) setting a weight for each position between the front wall boundary region and the rear wall boundary region to a preset maximum weight; (c4) setting a weight for each position within the rear wall boundary region based on a difference in ultrasound signal intensity between a start point and an end point of the rear wall boundary region; and (c5) setting a weight for positions after an end point of the rear wall boundary region to a preset minimum weight.

[ADVANTAGEOUS EFFECTS OF THE INVENTION]

The bladder volume measurement system according to the present invention extracts a region of interest from an ultrasound image using a region-of-interest extraction algorithm, and measures an area or a volume of the region of interest by using probability values and weights for all pixels constituting the ultrasound image. As described above, the bladder volume measurement system according to the present invention does not perform binarization, but instead measures the area or volume of the region of interest by using the probability values and weights for all pixels constituting the ultrasound image. Accordingly, sensitivity to a threshold setting is reduced, and measurement accuracy can be improved.

[BRIEF DESCRIPTION OF THE DRAWINGS]

FIG. 1 is an example of a bladder volume measurement system using an ultrasound scanner.

FIG. 2 is a two-dimensional ultrasound image acquired using the bladder volume measurement system of FIG. 1.

FIG. 3(a) is a two-dimensional ultrasound image acquired using a bladder volume measurement system, FIG. 3(b) illustrates a portion of probability values output for respective pixels of the two-dimensional ultrasound image shown in FIG. 3(a) using a machine learning algorithm, FIG. 3(c) illustrates a portion in which the probability values shown in FIG. 3(b) are converted into pixel values and FIG. 3(d) is an image reconstructed using the pixel values shown in FIG. 3(c).

FIG. 4(a) is a two-dimensional ultrasound image, FIG. 4(b) is an image reconstructed from probability values output by an extraction algorithm, FIG. 4(c) is an image binarized by setting a threshold to 0.5 and FIG. 4(d) is an image binarized by setting a threshold to 0.4.

FIG. 5 is a flowchart sequentially illustrating a method in which a controller measures an area or a volume of a bladder, which is a region of interest, in the bladder volume measurement system according to a first embodiment of the present invention.

FIG. 6 is a graph exemplarily illustrating a weighting function having a first threshold and a second threshold in the bladder volume measurement system according to the first embodiment of the present invention.

FIG. 7 illustrates a geometric model for a single pixel or voxel obtained in the bladder volume measurement system according to the first embodiment of the present invention.

FIG. 8 is a graph illustrating a bladder region for a single ultrasound reception signal in the bladder volume measurement system according to a second embodiment of the present invention.

[BEST MODE FOR CARRYING OUT THE INVENTION]

Hereinafter, a bladder volume measurement system and a bladder volume measurement method in the system according to preferred embodiments of the present invention will be described in detail with reference to the accompanying drawings.

<First Embodiment>

A bladder volume measurement system according to a first embodiment of the present invention includes an ultrasound probe and a controller. The ultrasound probe is configured to transmit and receive ultrasound signals for acquiring an ultrasound image by using an ultrasound transducer. The controller measures and provides a size of a region of interest included in the ultrasound signals received from the ultrasound probe. In the bladder volume measurement system according to the present invention, the region of interest measured by the controller refers to a bladder, and a volume of the bladder extracted and measured from the ultrasound signals represents an amount of urine filled in the bladder.

The ultrasound probe may include a plurality of transducers arranged in an array within the probe, or may include one or more transducers and motors for rotationally moving the transducer along a scan plane. That is, a plurality of transducers may be spaced apart at preset intervals and ultrasound signals may be sequentially acquired from the respective transducers, or ultrasound signals may be acquired while rotating the transducer sequentially by a preset angle using the motor. In general, the ultrasound transducer scans an object to be examined with ultrasound signals, and the scanned ultrasound signals are reflected from the object. The ultrasound probe acquires the ultrasound signals reflected from the object and provides the acquired ultrasound signals to the controller.

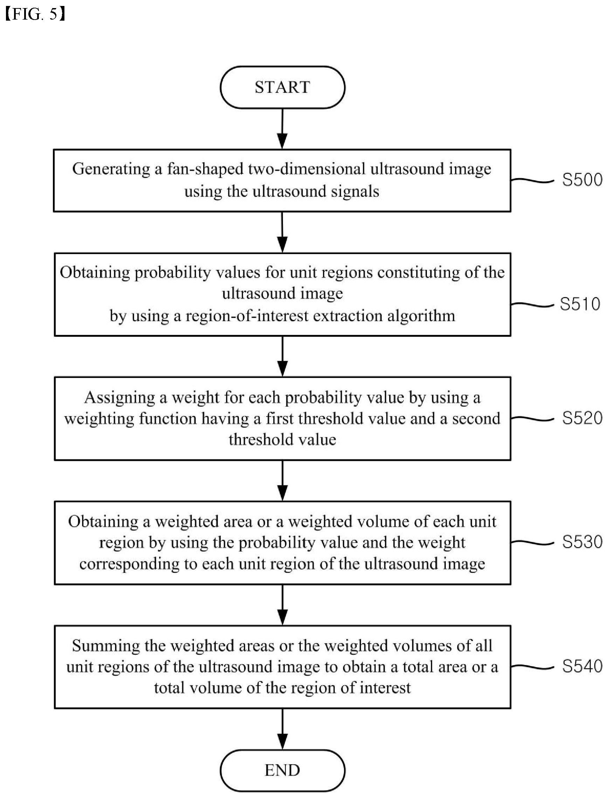

Hereinafter, a method in which the controller of the bladder volume measurement system according to the first embodiment of the present invention measures and provides a size of a region of interest included in ultrasound signals received from the ultrasound probe will be described in more detail. FIG. 5 is a flowchart sequentially illustrating a method in which the controller measures an area or a volume of the region of interest in the bladder volume measurement system according to the first embodiment of the present invention.

Referring to FIG. 5, the controller generates a fan-shaped two-dimensional ultrasound image using the ultrasound signals received from the ultrasound probe (step 500).

Next, by using a region-of-interest extraction algorithm such as a machine learning algorithm, a probability value that each unit region constituting the ultrasound image is included in the region of interest is obtained (step 510). Here, the unit regions may be composed of one or more pixels of the ultrasound image, or may be composed of one or more voxels.

Various methods may be used as the region-of-interest extraction algorithm. For example, the region-of-interest extraction algorithm may include: a Threshold Method that divides an image into a foreground and a background according to a single threshold; an Edge Detection Method that classifies edge pixels in an image and selects the edge pixels corresponding to a separate class based on the classified edge pixels; a Clustering-Based Method that starts from a seed pixel and then expands a region around the pixel according to a similarity criterion; and a Deep Learning-Based Method that trains features of an object in an image using a machine learning algorithm and then classifies segments as an object or a background using the trained machine learning algorithm.

In general, the above-described extraction algorithms may provide a probability value indicating a likelihood that a corresponding pixel or unit region is included in the region of interest. For example, if the likelihood that the corresponding pixel belongs to the region of interest is very high, the probability has a large value close to 1; and if the likelihood that the corresponding pixel does not belong to the region of interest and instead belongs to the background is very high, the probability has a small value close to 0. In particular, pixels near a boundary of the region of interest have both a possibility of belonging to the region of interest and a possibility of belonging to the background region. Accordingly, the probability value will have a value between 0 and 1, typically near 0.5. When the output of the region-of-interest extraction algorithm is not provided as a probability value, the output values may be normalized by a largest value among the output values, thereby converting the respective output values into probability values.

Next, in order to obtain a weight for each probability value, a preset weighting function is used to determine a weight (w) corresponding to a probability value (p) for each unit region constituting the ultrasound image (step 520). Here, the weighting function includes a first threshold (t1) and a second threshold (t2). A preset minimum weight is assigned to probability values (p) smaller than the first threshold, and a preset maximum weight is assigned to probability values greater than the second threshold. For probability values between the first threshold and the second threshold, weights are assigned linearly or nonlinearly within a range between the minimum weight and the maximum weight. The minimum weight may be set to "0," and the maximum weight may be set to "1".

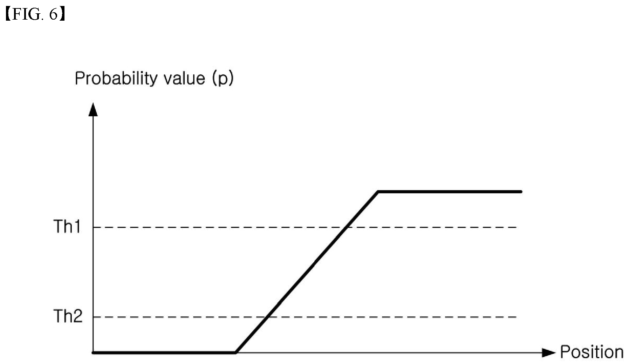

Here, the first threshold and the second threshold are values determined in consideration of characteristics of the system including noise, artifacts, and the like. Pixels having probability values less than or equal to the first threshold are regarded as belonging to a background region, and pixels having probability values greater than or equal to the second threshold are regarded as belonging to the region of interest. FIG. 6 is a graph exemplarily illustrating a weighting function having a first threshold (Th1) and a second threshold (Th2) in the bladder volume measurement system according to the first embodiment of the present invention.

An example of the weighting function may be expressed by Equation 1 below.

【EQUATION 1】

$$p < t1 \quad : \quad w = 0$$

$$t1 \leq p \leq t2 \quad : \quad w = (p-t1)/(t2-t1)$$

$$p > t2 \quad : \quad w = 1$$

In Equation 1, the weight is determined using a first-order linear expression. However, this is merely provided as an illustrative example. Accordingly, in consideration of system performance, various modified forms of mathematical expressions other than a first-order linear expression may also be used to determine the weight.

Next, a weighted area or a weighted volume for each unit region is obtained by multiplying a unit area or a unit volume of each unit region by the weight (w) for the corresponding unit region (step 530).

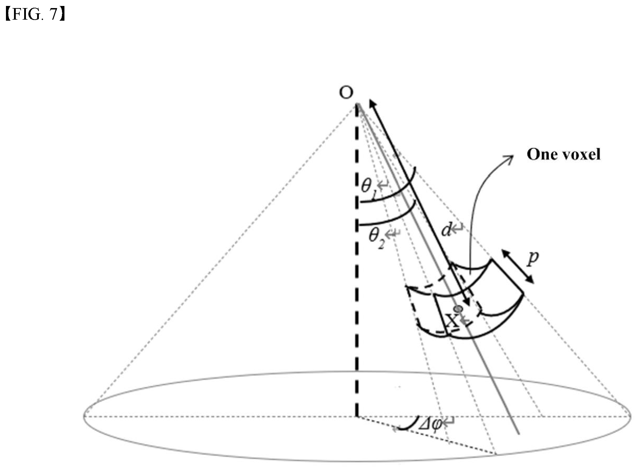

FIG. 7 illustrates a geometric model for a single pixel obtained in the bladder volume measurement system. Point O denotes a position of an ultrasound transducer of the bladder scanner, and point X denotes a center of a pixel in the acquired ultrasound image of the bladder scanner. In the ultrasound image of the bladder scanner, one pixel corresponds to one voxel indicated by a thick line in the figure. Here, $p$ denotes a pixel size in a depth direction, and $d$ denotes a distance from point O to the pixel center X. $\Delta\varphi$ denotes an angle between ultrasound cross-sectional images obtained by the bladder scanner, and $\theta_1$ and $\theta_2$ denote scan angles of ultrasound scan lines of the bladder volume measurement system. A volume $\Delta V'$ of this pixel (voxel) may be calculated by Equation 2 for calculating a volume of a quadrangular pyramid.

【EQUATION 2】

$$\Delta V' = \int_{d-p/2}^{d+p/2} \int_{\theta_2}^{\theta_1} \int_0^{\Delta\varphi} r^2 \sin\theta \, d\varphi d\theta dr = \frac{1}{3}\{(d+p/2)^3 - (d-p/2)^3\}\Delta\varphi(\cos\theta_2$$

$$- \cos\theta_1)$$

In the proposed method, a weighted volume ΔV of the voxel is obtained by multiplying the volume ΔV' by the weight (w) calculated above.

【EQUATION 3】

$$\Delta V = w \cdot \Delta V'$$

Next, the total area or the total volume of the region of interest is finally calculated by summing all the weighted areas or the weighted volumes for the respective unit regions (step 540).

The first threshold and the second threshold set in the present embodiment affect the calculation of the area or volume of the region of interest. However, compared with the conventional technique that separates a background region and a region of interest by a binarization method, the method according to the present embodiment exhibits significantly lower sensitivity to the thresholds. This is because, in calculating the area or volume of the region of interest, the method according to the present embodiment applies weights to probability values of unit regions located at a boundary between the background region and the region of interest, in consideration of factors such as a distance from a center of the region of interest. Accordingly, in the bladder volume measurement method according to the present embodiment, even if the first threshold and the second threshold of the weighting function are slightly changed, a final result value does not change significantly.

However, in the case of using the binarization method according to the conventional technique, a single threshold is used to separate the region of interest from the background region. Accordingly, when the binarization method according to the conventional technique is used, a final result value for calculating the total area or total volume is highly sensitive to the threshold. For example, in the conventional technique using the binarization method, when the threshold is set to 0.5, a pixel having a probability value of 0.51 is included in the region of interest and weighted as 1, thereby being fully reflected (100%), whereas a pixel having a probability value of 0.49 is weighted as 0 and is not reflected at all. In contrast, in the present embodiment, all pixels having probability values of 0.51 and 0.49 are weighted and applied in the area or volume calculation. Accordingly, pixels located at an ambiguous boundary between the region of interest and the background region can also be reflected in the calculation.

Performance of a method for calculating a volume using probability values and weights for all pixels according to the present invention and a method for calculating a volume using binarization according to the conventional technique will now be examined. In order to compare the performance of the method according to the present invention with that of the conventional method, the ultrasound image of FIG. 2 was used. Using 12 ultrasound images including the image shown in FIG. 2, volumes of the bladder, which is the region of interest, were calculated according to the method of the present invention and according to the conventional method. As a result, outcomes as shown in the table below were obtained.

[TABLE1]

| Method | Threshold | Volume | Difference |
|---|---|---|---|
| Conventional | 0.5 | 161ml | 16ml, 9.1% |
| Method | 0.4 | 176ml | |
| Present | 0.1~0.9 (Th1~Th2) | 170ml | 1ml, 0.6% |
| Invention | 0.15~0.85(Th1~Th2) | 169ml | |

Here, in the conventional method, the volume was calculated by setting the threshold to 0.4 and 0.5, respectively.

When the threshold was set to 0.5, the volume was 161 ml, and when the threshold was set to 0.4, the volume was 176 ml. The difference therebetween was 16 ml, corresponding to 9.1%. In contrast, according to the method of the present invention, when the first threshold and the second threshold were set to 0.1 and 0.9, respectively, the volume was 170 ml; and when the first threshold and the second threshold were set to 0.15 and 0.85, respectively, the volume was 169 ml. The difference therebetween was 1 ml, corresponding to 0.6%. From these measurements, it can be seen that the conventional technique using the binarization method is highly sensitive to the threshold setting, and thus has a high possibility of causing errors. In contrast, the method according to the present invention exhibits little sensitivity to the threshold setting and provides high reliability.

[MODE FOR CARRYING OUT THE INVENTION]

<Second Embodiment>

Hereinafter, a bladder volume measurement system according to a second embodiment of the present invention will be described in detail. The bladder volume measurement system according to the second embodiment of the present invention has the same structure as the bladder volume measurement system according to the above-described first embodiment, but differs in a region-of-interest extraction algorithm used by the controller. Accordingly, a bladder volume measurement method performed by the controller of the bladder volume measurement system according to the second embodiment of the present invention will be described in more detail.

The bladder volume measurement method according to the second embodiment of the present invention is characterized in that an ultrasound signal is analyzed to detect a front wall point and a rear wall point of a bladder, and a volume of a bladder region is measured by applying weights to regions surrounding the detected points. In a conventional technique, positions of the front wall and the rear wall of the bladder are detected by analyzing an intensity of the ultrasound signal, and an area between the detected front wall position and rear wall position is regarded entirely as the bladder region, while other areas are regarded as background regions to measure a size of the bladder. However, when the front wall and rear wall positions are detected according to such a conventional technique, the detected positions may vary very sensitively depending on a threshold, and as a result, the measured bladder volume may also vary significantly. In consideration of these problems, the bladder volume measurement method in the bladder volume measurement system according to the present embodiment is characterized in that an intensity of the ultrasound signal is analyzed to detect a front wall point and a rear wall point of the bladder, a front wall boundary region and a rear wall boundary region are set for regions surrounding the detected front wall point and rear wall point, respectively, and a volume of the bladder region is measured by applying weights to respective positions within the front wall boundary region and the rear wall boundary region.

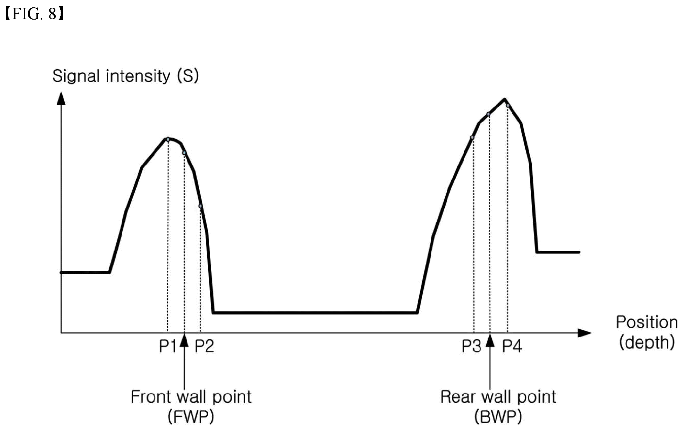

Hereinafter, the bladder volume measurement method in the bladder volume measurement system according to the second embodiment of the present invention will be described in more detail. FIG. 8 is a graph illustrating a bladder region with respect to a single ultrasound signal in the bladder volume measurement system according to the second embodiment of the present invention.

First, a front wall point (FWP) and a rear wall point (BWP) of the bladder are detected using an intensity of the ultrasound signal.

Next, a preset front-and-rear interval (P1 to P2) with respect to the front wall point (FWP) of the bladder is included to define a front wall boundary region of the bladder, and a preset front-and-rear interval (P3 to P4) with respect to the rear wall point (BWP) of the bladder is included to define a rear wall boundary region of the bladder.

Next, with reference to the front wall boundary region and the rear wall boundary region, a weight (w) is set for each position (p) of the ultrasound signal. One embodiment of a method for setting the weight for each position is as follows.

For positions prior to a start point of the front wall boundary region (p < P1), the weight (w) is set to a minimum weight. Here, the minimum weight may be set to "0" (w = 0).

For each position within the front wall boundary region (P1 < p < P2), the weight (w) for each position in the front wall boundary region is set according to Equation 4, based on a difference between ultrasound signal intensities (S1, S2) at a start point (P1) and an end point (P2) of the front wall boundary region.

【EQUATION 4】

$$w = (S_p - S_1)/(S_2 - S_1)$$

For each position between the front wall boundary region and the rear wall boundary region (P2 < p < P3), the weight (w) is set to a maximum weight. Here, the maximum weight may be set to "1" (w = 1).

For each position within the rear wall boundary region (P3 < p < P4), the weight (w) for each position in the rear wall boundary region is set according to Equation 5, based on a difference between ultrasound signal intensities (S3, S4) at a start point (P3) and an end point (P4) of the rear wall boundary region.

【EQUATION 5】

$$w = (S_p - S_3)/(S_4 - S_3)$$

For positions after the end point (P4) of the rear wall boundary region (p > P4), the weight (w) is set to the minimum weight. Here, the minimum weight may be set to "0" (w = 0).

Through the above-described process, a weight for each position may be set.

Based on detection of the front wall and the rear wall of the bladder from the ultrasound signal and weight information of adjacent regions determined accordingly, a volume may be calculated in the same manner as in FIG. 7 by combining information of a plurality of ultrasound scan lines forming a cross-section and information of a plurality of cross-sections.

Although the present invention has been described above with reference to preferred embodiments thereof, these embodiments are merely illustrative and are not intended to limit the present invention. It will be apparent to those skilled in the art that various modifications and applications not exemplified above may be made without departing from the essential characteristics of the present invention. Accordingly, differences relating to such modifications and applications should be construed as being included within the scope of the present invention as defined by the appended claims.

**Claims**

1.  A bladder volume measurement system comprising:

    an ultrasound probe configured to receive ultrasound signals for acquiring an ultrasound image by using an ultrasound transducer; and
    a controller configured to measure and provide an area of a region of interest included in the ultrasound signals received from the ultrasound probe,
    wherein the controller
    generates the ultrasound image by using the ultrasound signals received from the ultrasound probe,
    infers, by using a predetermined region-of-interest extraction algorithm, a probability value that each unit region constituting the ultrasound image is included in the region of interest,
    determines weights by using a preset weighting function according to a distribution of the probability values, and
    calculates and provides an area or a volume of the region of interest by using the probability values and the weights for all unit regions constituting the ultrasound image.

2.  The bladder volume measurement system according to claim 1, wherein the controller determines weights for probability values of unit regions constituting the ultrasound image by using the weighting function, obtains a weighted area or a weighted volume for each unit region by multiplying a unit area or a unit volume of each unit region constituting the ultrasound image by the weight of the corresponding unit region, and calculates the area or the volume of the region of interest by summing the weighted areas or the weighted volumes of all unit regions constituting the ultrasound image.

3.  The bladder volume measurement system according to claim 1, wherein the weighting function includes a first threshold and a second threshold, assigns a preset minimum weight to probability values smaller than the first threshold, assigns a preset maximum weight to probability values greater than the second threshold, and assigns weights linearly or nonlinearly within a range between the minimum weight and the maximum weight to probability values between the first threshold and the second threshold.

4.  The bladder volume measurement system according to claim 3, wherein the minimum weight is "0" and the maximum weight is "1".

5.  The bladder volume measurement system according to claim 1, wherein the region-of-interest extraction algorithm extracts the region of interest from the ultrasound image by using a machine learning algorithm.

**6.** A bladder volume measurement method performed by a controller of a bladder volume measurement system configured to measure and provide a volume of a bladder using ultrasound signals, the method comprising the following steps:

(a) generating an ultrasound image by using ultrasound signals received from an ultrasound probe;
(b) inferring, by using a preset region-of-interest extraction algorithm, a probability value that each unit region constituting the ultrasound image is included in a region of interest; and
(c) determining weights by using a preset weighting function according to a distribution of the probability values for the respective unit regions, and calculating and providing an area or a volume of the region of interest by using the probability values and the weights for all unit regions constituting the ultrasound image.

**7.** The bladder volume measurement method according to claim 6, wherein step (c) comprises:

(c1) determining weights for probability values of unit regions constituting the ultrasound image by using the weighting function;
(c2) obtaining a weighted area or a weighted volume for each unit region by multiplying a unit area or a unit volume of each unit region constituting the ultrasound image by the weight of the corresponding unit region; and
(c3) calculating the area or the volume of the region of interest by summing the weighted areas or the weighted volumes of all unit regions constituting the ultrasound image.

**8.** The bladder volume measurement method according to claim 6, wherein the weighting function includes a first threshold and a second threshold, assigns a preset minimum weight to probability values smaller than the first threshold, assigns a preset maximum weight to probability values greater than the second threshold, and assigns weights linearly or nonlinearly within a range between the minimum weight and the maximum weight to probability values between the first threshold and the second threshold.

**9.** A bladder volume measurement system comprising:

an ultrasound probe configured to receive ultrasound signals for acquiring an ultrasound image by using an ultrasound transducer; and
a controller configured to measure and provide an area of a region of interest included in the ultrasound signals received from the ultrasound probe,
wherein the controller
analyzes the ultrasound signals received from the ultrasound probe to detect a front wall point and a rear wall point of a bladder,
sets a front wall boundary region including preset front-and-rear regions of the front wall point of the bladder and sets a rear wall boundary region including preset front-and-rear regions of the rear wall point of the bladder,
assigns a weight for each position of the ultrasound signal based on the front wall boundary region and the rear wall boundary region, and
calculates and provides an area or a volume of a bladder region by using the weights for the respective positions of the ultrasound signal.

**10.** The bladder volume measurement system according to claim 9, wherein the controller

sets a weight for a position prior to a start point of the front wall boundary region to a preset minimum weight,
sets a weight for each position within the front wall boundary region based on a difference between ultrasound signal intensities at a start point and an end point of the front wall boundary region,
sets a weight for each position between the front wall boundary region and the rear wall boundary region to a preset maximum weight,
sets a weight for each position within the rear wall boundary region based on a difference between ultrasound signal intensities at a start point and an end point of the rear wall boundary region, and
sets a weight for a position after an end point of the rear wall boundary region to the minimum weight.

**11.** The bladder volume measurement system according to claim 9, wherein the region-of-interest extraction algorithm extracts the region of interest from the ultrasound image by using a machine learning algorithm.

**12.** A bladder volume measurement method performed by a controller of a bladder volume measurement system configured to measure and provide a volume of a bladder using ultrasound signals, the method comprising the following steps:

(a) analyzing ultrasound signals received from an ultrasound probe to detect a front wall point and a rear wall point of the bladder;

(b) setting a front wall boundary region including a preset front-and-rear region of the front wall point of the bladder, and setting a rear wall boundary region including a preset front-and-rear region of the rear wall point of the bladder;

(c) assigning a weight for each position of the ultrasound signal based on the front wall boundary region and the rear wall boundary region; and

(d) calculating and providing an area or a volume of a bladder region by using the weights for the respective positions of the ultrasound signal.

**13.** The bladder volume measurement method according to claim 12, wherein step (c) comprises:

(c1) setting a weight for a position prior to a start point of the front wall boundary region to a preset minimum weight;

(c2) setting a weight for each position within the front wall boundary region based on a difference between ultrasound signal intensities at a start point and an end point of the front wall boundary region;

(c3) setting a weight for each position between the front wall boundary region and the rear wall boundary region to a preset maximum weight;

(c4) setting a weight for each position within the rear wall boundary region based on a difference between ultrasound signal intensities at a start point and an end point of the rear wall boundary region; and

(c5) setting a weight for a position after an end point of the rear wall boundary region to the minimum weight.

【FIG. 1】

【 FIG. 2】

【FIG. 3】

(a)

(b)

(c)

(d)

【FIG. 4】

(a)          (b)          (c)          (d)

【FIG. 5】

```
                    ┌─────────────┐
                    │    START    │
                    └─────────────┘
                           │
                           ▼
  ┌────────────────────────────────────────────────┐
  │ Generating a fan-shaped two-dimensional         │ ──── S500
  │ ultrasound image using the ultrasound signals   │
  └────────────────────────────────────────────────┘
                           │
                           ▼
  ┌────────────────────────────────────────────────┐
  │ Obtaining probability values for unit regions   │
  │ constituting of the ultrasound image            │ ──── S510
  │ by using a region-of-interest extraction        │
  │ algorithm                                       │
  └────────────────────────────────────────────────┘
                           │
                           ▼
  ┌────────────────────────────────────────────────┐
  │ Assigning a weight for each probability value   │
  │ by using a weighting function having a first    │ ──── S520
  │ threshold value and a second threshold value    │
  └────────────────────────────────────────────────┘
                           │
                           ▼
  ┌────────────────────────────────────────────────┐
  │ Obtaining a weighted area or a weighted volume  │
  │ of each unit region by using the probability    │ ──── S530
  │ value and the weight corresponding to each      │
  │ unit region of the ultrasound image             │
  └────────────────────────────────────────────────┘
                           │
                           ▼
  ┌────────────────────────────────────────────────┐
  │ Summing the weighted areas or the weighted      │
  │ volumes of all unit regions of the ultrasound   │ ──── S540
  │ image to obtain a total area or a total volume  │
  │ of the region of interest                       │
  └────────────────────────────────────────────────┘
                           │
                           ▼
                    ┌─────────────┐
                    │     END     │
                    └─────────────┘
```

【FIG. 6】

【FIG. 7】

【FIG. 8】

# INTERNATIONAL SEARCH REPORT

| | International application No. |
|---|---|
| | **PCT/KR2023/014572** |

## A. CLASSIFICATION OF SUBJECT MATTER

**A61B 8/08**(2006.01)i; **G06T 7/62**(2017.01)i

According to International Patent Classification (IPC) or to both national classification and IPC

## B. FIELDS SEARCHED

Minimum documentation searched (classification system followed by classification symbols)

A61B 8/08(2006.01); A61B 8/00(2006.01); G06T 7/00(2006.01); G06T 7/62(2017.01); G06V 10/62(2022.01); G06V 20/40(2022.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 초음파(ultrasound), 방광(bladder), 프로브(probe), 단위 영역(unit area), 관심 영역 (region of interest), 확률(probability), 가중(weight)

## C. DOCUMENTS CONSIDERED TO BE RELEVANT

| Category* | Citation of document, with indication, where appropriate, of the relevant passages | Relevant to claim No. |
|---|---|---|
| A | KR 10-2023-0008984 A (MCUBETECHNOLOGY CO., LTD.) 17 January 2023 (2023-01-17) See claims 1 and 2. | 1-13 |
| A | JP 2010-527277 A (VERATHON INC.) 12 August 2010 (2010-08-12) See paragraphs [0071] and [0128]. | 1-13 |
| A | JP 2014-138847 A (SIEMENS CORP.) 31 July 2014 (2014-07-31) See claims 1-18. | 1-13 |
| A | CN 110991254 B (SHENZHEN UNIVERSITY) 04 July 2023 (2023-07-04) See entire document. | 1-13 |
| A | KR 10-2181137 B1 (DAEGU GYEONGBUK INSTITUTE OF SCIENCE AND TECHNOLOGY et al.) 20 November 2020 (2020-11-20) See entire document. | 1-13 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2024** | **10 June 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/014572**

| Patent document cited in search report | | | Publication date (day/month/year) | Patent family member(s) | | | Publication date (day/month/year) |
|---|---|---|---|---|---|---|---|
| KR | 10-2023-0008984 | A | 17 January 2023 | | None | | |
| JP | 2010-527277 | A | 12 August 2010 | CA | 2688758 | A1 | 27 November 2008 |
| | | | | CA | 2688758 | C | 05 July 2016 |
| | | | | CA | 2688778 | A1 | 27 November 2008 |
| | | | | CA | 2688778 | C | 14 April 2020 |
| | | | | EP | 2155067 | A2 | 24 February 2010 |
| | | | | EP | 2155067 | A4 | 23 June 2010 |
| | | | | EP | 2155067 | B1 | 06 January 2016 |
| | | | | EP | 2155068 | A1 | 24 February 2010 |
| | | | | EP | 2155068 | A4 | 16 June 2010 |
| | | | | EP | 2155068 | B1 | 21 December 2016 |
| | | | | JP | 2010-527278 | A | 12 August 2010 |
| | | | | JP | 2015-042279 | A | 05 March 2015 |
| | | | | JP | 5632278 | B2 | 26 November 2014 |
| | | | | JP | 6162094 | B2 | 12 July 2017 |
| | | | | US | 2009-0105585 | A1 | 23 April 2009 |
| | | | | WO | 2008-144449 | A2 | 27 November 2008 |
| | | | | WO | 2008-144449 | A3 | 30 December 2009 |
| | | | | WO | 2008-144452 | A1 | 27 November 2008 |
| JP | 2014-138847 | A | 31 July 2014 | CN | 103908300 | A | 09 July 2014 |
| | | | | CN | 103908300 | B | 24 April 2018 |
| | | | | DE | 102013021729 | A1 | 03 July 2014 |
| | | | | JP | 6230917 | B2 | 15 November 2017 |
| | | | | KR | 10-1907550 | B1 | 12 October 2018 |
| | | | | KR | 10-2014-0088840 | A | 11 July 2014 |
| | | | | US | 2014-0187942 | A1 | 03 July 2014 |
| | | | | US | 9642592 | B2 | 09 May 2017 |
| CN | 110991254 | B | 04 July 2023 | CN | 110991254 | A | 10 April 2020 |
| KR | 10-2181137 | B1 | 20 November 2020 | EP | 3973886 | A2 | 30 March 2022 |
| | | | | US | 2022-0183660 | A1 | 16 June 2022 |
| | | | | WO | 2020-235810 | A2 | 26 November 2020 |
| | | | | WO | 2020-235810 | A3 | 07 January 2021 |

Form PCT/ISA/210 (patent family annex) (July 2022)